# DEMANDE DE BREVET EUROPEEN

(11) **EP 4 062 930 A1**
(43) Date de publication de la demande: **28.09.2022**
(21) Numéro de dépôt: 21180476.0
(22) Date de dépôt: 18.06.2021
(51) Int. Cl.: A61K 39/215

(54) **NOUVELLE APPLICATION D'UNE COMPOSITION IMMUNOGENE OU VACCINALE CONTRE LA COVID-19**

(30) Priorité: 22.03.2021 DE 102021001467
(71) Demandeur: EYE VACC, 4031 Liège (Angleur) (BE)
(72) Inventeur: SCHRAGE, Norbert, D-52070 Aix-La-Chapelle (DE); HOLZER, Frank, 66129 Saarbrücken (DE); BLOMET, Joël, 75015 Paris (FR); DESMECHT, Daniel, B-4141 Louveigne (BE)
(74) Mandataire: Plasseraud IP

(57) **Abrégé**

La présente invention concerne une composition immunogène ou vaccinale contre le SARS-CoV-2, pour son utilisation dans le traitement de la Covid-19, caractérisée en ce qu'elle est administrée sur la muqueuse oculaire et/ou la muqueuse uro-génitale.

## Description

En plus du système immunitaire systémique, notre organisme contient un système immunitaire associé aux muqueuses (également dénommé MALT, Mucosa Associated Lymphoid Tissue). Les muqueuses contiennent localement leur propre système immunitaire, par exemple au niveau du tractus digestif (GALT, Gut Associated Lymphoid Tissue), au niveau du nez (NALT, Nasal Associated Lymphoid Tissue), ou encore au niveau de l'œil (CALT, Conjunctiva Associated Lymphoid Tissue

En effet, les sites d'entrée des agents pathogènes dans l'organisme se situent généralement au niveau des muqueuses de l'œil, du nez, de la bouche ou du tractus gastro-intestinal. Notre organisme contient ainsi un grand nombre de mécanismes de défense cellulaires et biochimiques, directement au niveau de ces muqueuses, qui s'activent au contact des agents pathogènes. Typiquement le système immunitaire des muqueuses comprend les cellules épithéliales, les cellules de l'immunité innée et les cellules dendritiques qui sont à l'interface entre l'immunité inné et l'immunité spécifique (acquise). Les muqueuses peuvent ainsi contenir des cellules de l'immunité innée, des cellules immunocompétentes, des cellules mémoires et des cellules productrices d'anticorps.

Après avoir survécu à une primo-infection par un agent pathogène, l'organisme développe une immunité systémique (sérique) contre cet agent. Par ailleurs, si l'agent pathogène a été en contact avec les muqueuses de l'organisme, une immunité des muqueuses est également développée, en plus de l'immunité systémique.

Des vaccins permettant de développer l'immunité des muqueuses sont déjà connus. Ces derniers sont administrés essentiellement par voie nasale ou orale. Ils offrent l'avantage singulier d'induire une réponse immunitaire protectrice, tant au niveau des muqueuses qu'au niveau systémique. Ils visent également à empêcher le franchissement des muqueuses, porte d'entrée de la plupart des agents pathogènes bactériens et viraux. C'est par exemple le cas du vaccin contre la grippe FluMist^{®} qui est administré par vaporisation nasale, ou encore les vaccins contre la poliomyélite qui s'administrent par voie orale. La vaccination contre un virus de la grippe (virus influenza A), par voie oculaire, chez des furets a également été étudiée dans l'article Eyedrop Vaccination Induced Systemic and Mucosal Immunity against Influenza Virus in Ferrets de Sangchul Yoon, Eun-Do Kim, Min-Suk Song, Soo Jung Han, Tae Kwann Park, Kyoung Sub Choi, Young-Ki Choi, et Kyoung Yul Seo; PLoS One. 2016; 11(6): e0157634. La vaccination par voie oculaire a également été étudiée chez des souris par l'équipe Kyoung Yul Seo, Soo Jung Han, Hye-Ran Cha, Sang-Uk Seo, Joo-Hye Song, So-Hyang Chung, et Mi-Na Kweon (Eye Mucosa: An Efficient Vaccine Delivery Route for Inducing Protective Immunity; J Immunol 2010; 185:3610-3619), bien que les souris ne soient pas des hôtes naturels du virus.

Dans le cas d'agents pathogènes colonisant les muqueuses et hautement contagieux, l'agent pathogène se multiplie si rapidement après avoir colonisé les muqueuses que l'infection des muqueuses et des couches plus profondes progresse plus vite que l'organisme ne peut établir une immunité efficace pour éliminer le pathogène. Ceci est typiquement le cas de l'infection actuelle par le coronavirus 2 du syndrome respiratoire aigu sévère (SRAS-CoV-2 ou Severe Acute Respiratory Syndrome CoronaVirus 2). En effet, la Covid-19 (ou maladie à coronavirus 2019) présente principalement une infection et une inflammation des muqueuses avec une maladie générale grave secondaire (comme cela est également le cas pour la grippe, l'herpès ou la poliomyélite).

La pandémie actuelle de la Covid-19 a donné lieu à une recherche sans précédent, notamment afin de développer des vaccins, et à ce jour, plusieurs vaccins anti-Covid-19 ont déjà été autorisés pour vacciner la population mondiale. De tels vaccins sont par exemple (1) le vaccin à ARNm Pfizer/BioNTech, généralement dénommé Bnt162b2 ou Comirnaty^{®} ; (2) le vaccin à ARNm de Moderna, généralement dénommé Moderna mRNA-1273 ou COVID-19 Vaccine Moderna; (3) le vaccin à vecteur viral non réplicatif d'AstraZeneca, généralement dénommé Vaxzevria^{®}, ChAdOx1-S ou COVID-19 Vaccine AstraZeneca ; (4) le vaccin à vecteur viral non réplicatif de Janssen, généralement dénommé Ad26COV2.S, JMJ Vaccine ou J & J COVID-19 ou encore (5) le vaccin à vecteur viral non réplicatif de Gamaleya, généralement dénommé Spoutnik V ou Gam-COVID-Vac.

Tous les vaccins contre la Covid-19 actuellement disponibles sont administrés par voie intramusculaire. Ils engendrent ainsi une bonne immunité systémique, permettant de limiter les évolutions sévères de la maladie, mais ils ne permettent pas de développer l'immunité des muqueuses. Cette immunité des muqueuses est cependant essentielle à la prévention de nouvelles infections, faute de quoi le risque d'infection au SRAS-CoV-2 restera élevé.

Par ailleurs, les quantités de vaccins actuellement disponibles restent encore trop faibles, et les éventuels risques secondaires de ces vaccins (tels que les thromboses) sont également une source d'inquiétude pour certaines personnes, qui hésitent ainsi à se faire vacciner.

Il existe donc toujours un besoin pour améliorer la vaccination de la population mondiale contre la Covid-19, permettant ainsi la sortie de cette crise pandémique.

La présente invention répond à ce besoin grâce à un nouveau mode de vaccination contre la Covid-19 qui permet une amélioration de l'immunité (la double immunité -systémique et des muqueuses- assure en effet une meilleure protection vis-à-vis de l'agent pathogène, et ce faisant contribue au développement de l'immunité collective), tout en diminuant les risques d'effet secondaires graves liés à la vaccination.

La présente invention vise aussi à fournir un nouveau mode de vaccination contre la Covid-19, qui soit plus simple à mettre en œuvre et plus rapide, ceci afin d'augmenter les rythmes de vaccination.

La présente invention permet de développer une immunité des muqueuses contre le SRAS-CoV-2, en ciblant la muqueuse oculaire et/ou la muqueuse uro-génitale.

La présente invention concerne ainsi une composition immunogène ou vaccinale contre le SARS-CoV-2, pour son utilisation dans la prévention et/ou le traitement de la Covid-19, caractérisée en ce qu'elle est administrée sur la muqueuse oculaire et/ou la muqueuse uro-génitale.

L'intérêt de l'administration d'une telle composition est d'établir à la fois une immunité des muqueuses et une immunité sérique contre le SRAS-CoV-2. Cette double immunité permet de mieux protéger l'organisme vis-à-vis du virus et limite le risque d'infections, sans subir les graves effets secondaires systémiques qui peuvent être constatés avec certains vaccins actuellement injectés par voie intramusculaire (notamment les thromboses). En effet, dans le cas de la présente invention, étant donné que l'immunité sérique est acquise indirectement *via* une immunisation des muqueuses, un accident thrombotique n'est donc pas attendu.

L'expression « composition immunogène » et/ou « composition vaccinale » selon l'invention s'entend d'une composition qui induit une réponse immunitaire contre le SRAS-CoV-2 après administration chez le sujet. Une composition vaccinale permet notamment de générer une immunité, plus particulièrement une réponse immunitaire protectrice et adaptative contre le SRAS-CoV-2. Cette réponse immunitaire peut être humorale et/ou cellulaire.

Le terme « SARS-CoV-2 » selon l'invention s'entend du virus appartenant à la famille des Coronaviridae, au genre Betacoronavirus, et au sous-genre Sarbecovirus. Il s'agit d'un virus enveloppé à capside hélicoïdale dont le génome est constitué d'ARN simple brin d'environ 30 000 nucléotides. La structure du virion de SARS-CoV-2 est la suivante : il contient une capside hélicoïdale formée de protéine N, une matrice formée de protéine M et une enveloppe lipidique dans laquelle trois types de protéines sont enchâssés : la (glyco)protéine S (spike), la petite protéine d'enveloppe (E) et l'hémagglutinine-estérase (HE). La protéine S est la principale cible de la réponse immunitaire par production d'anticorps neutralisants. Cette protéine de surface se lie au récepteur ACE2 (qui est exprimé dans de nombreux tissus), ce qui permet au virus de pénétrer dans les cellules de l'organisme contaminé. La protéine S contient deux sous-unités, S1 et S2. S1 inclut le domaine de liaison au récepteur (RBD, Receptor Binding Domain) qui contient le motif de liaison au récepteur (RBM, Receptor Binding Motif). La sous-unité S2 contient quant à elle un peptide de fusion qui permet la fusion entre la membrane cellulaire de la cellule hôte (de l'organisme contaminé) et l'enveloppe virale.

Plus particulièrement, le terme « SARS-CoV-2 » selon l'invention s'entend du virus dont la séquence a été initialement décrite dans GenBank, sous le code d'accession MN908947, ainsi que tous les variants de ce virus, notamment le variant dit anglais, le variant dit sud-africain, le variant dit indien ou le variant dit brésilien/japonais. Des informations concernant les séquences du SARS-CoV-2, les mutations et les séquences des variants peuvent être trouvées, par exemple, aux liens suivants : https://www.ncbi.nlm.nih.gov/sars-cov-2/ ; https://www.cdc.gov/coronavirus/2019-ncov/cases-updates/variant-surveillance/variant-info.html ; ou encore https://www.gisaid.org/.

L'expression « la prévention de la Covid-19 » selon l'invention s'entend de la prophylaxie. L'administration de la composition immunogène ou vaccinale selon l'invention permet notamment d'empêcher ou de réduire le risque de développer la Covid-19, et/ou réduit le risque de développer des formes dites sévères de la maladie (c'est-à-dire avec des symptômes graves tels que des difficultés à respirer, et/ou nécessitant une hospitalisation, bien souvent en service de réanimation). L'administration de la composition immunogène ou vaccinale selon l'invention pourrait également empêcher ou diminuer le risque de transmission du virus, typiquement d'une personne à une autre. Selon un mode de réalisation particulier, la composition immunogène ou vaccinale pour la prévention de la Covid-19 selon l'invention est administrée chez un sujet n'étant pas contaminé, de préférence n'étant pas infecté, par le SARS-CoV-2.

L'expression «le traitement de la Covid-19» selon l'invention s'entend de la thérapie. L'administration de la composition immunogène ou vaccinale selon l'invention peut en effet être envisagée, afin de stimuler les défenses naturelles de l'organisme, alors même que la personne est *a minima* déjà contaminée par le virus. Selon un mode de réalisation particulier, la composition immunogène ou vaccinale pour le traitement de la Covid-19 selon l'invention est administrée chez un sujet étant contaminé, et/ou étant infecté, par le SARS-CoV-2.

De préférence, la présente invention concerne une composition immunogène ou vaccinale contre le SARS-CoV-2, pour son utilisation dans la prévention de la Covid-19, caractérisée en ce qu'elle est administrée sur la muqueuse oculaire et/ou la muqueuse uro-génitale.

L'expression « la muqueuse oculaire » selon l'invention s'entend de la conjonctive et de la cornée. La conjonctive est la membrane muqueuse transparente qui recouvre la face interne des paupières supérieures et inférieures et qui couvre la surface antérieure du globe oculaire. Plus précisément, la muqueuse oculaire s'entend donc à la fois de la conjonctive tarsale et de la conjonctive bulbaire, de même que les culs-de-sac conjonctivaux.

L'expression « la muqueuse uro-génitale » selon l'invention s'entend des muqueuses de l'appareil urinaire et/ou génital, tant l'appareil masculin que l'appareil féminin. Plus précisément, la muqueuse uro-génitale s'entend donc du tractus uro-génital.

Le ciblage de la muqueuse oculaire et/ou la muqueuse uro-génitale permet de cibler des muqueuses qui (i) ne participent en elles-mêmes que dans une faible mesure à la diffusion de l'agent pathogène, mais (ii) qui présentent en même temps une immunocompétence très élevée, qui permet d'immuniser très rapidement l'organisme, notamment avant une infection des voies respiratoires.

Une des hypothèses, non limitative, des inventeurs est en effet qu'une infection de l'œil par le SRAS-CoV-2 entraîne la formation rapide d'une immunité, permettant à l'organisme de gagner du temps, de façon à ce que lorsque la contamination et/ou l'infection progresse vers le nez, l'organisme est déjà immunisé. Cette progression de la contamination et/ou l'infection de l'œil vers le nez pourrait s'expliquer grâce à la présence du canal nasolacrimal qui relie le nez aux yeux. Une infection par le canal nasolacrimal a déjà été décrite dans le cas de kératite causée par des virus (de type *keratitis epidemica*). L'évolution typique de cette maladie commence par une infection de la conjonctive par des gouttelettes et se manifeste par une conjonctivite sévère suivie d'une pharyngite. Cela pourrait également expliquer pourquoi des sujets, notamment du personnel médical, ont développé une immunité systémique contre le SRAS-CoV-2 après avoir développé une conjonctivite positive au SRAS-CoV-2.

De préférence, ladite composition immunogène ou vaccinale est administrée sur la muqueuse oculaire.

Selon un mode de réalisation encore plus préféré, la présente invention concerne une composition immunogène ou vaccinale contre le SARS-CoV-2, pour son utilisation dans la prévention de la Covid-19, caractérisée en ce qu'elle est administrée sur la muqueuse oculaire.

Selon l'invention, ladite composition immunogène ou vaccinale peut être administrée sur un seul ou sur les deux yeux, de préférence sur les deux yeux. Contrairement à une administration intramusculaire d'un vaccin qui nécessite une organisation et un effort médical accru (*e.g.* préparation des seringues, nécessité de disposer d'un équipement de réanimation en cas de réaction grave à la vaccination, ...), l'injection sur une muqueuse oculaire, typiquement par collyre, est bien plus simple et rapide à mettre en œuvre. En effet, les risques graves sont diminués (par exemple les risques de thrombose comme indiqués ci-dessus), de même que les graves risques allergiques qui se manifestent principalement par des picotements aux yeux, un larmoiement ou encore des yeux rouges. La vaccination par collyre permet par ailleurs de vacciner bien plus rapidement et à plus grande échelle.

Selon un mode de réalisation, ladite composition immunogène ou vaccinale comprend une ou plusieurs substances permettant de provoquer une réaction immunitaire contre le SARS-CoV-2. Typiquement, ladite composition immunogène ou vaccinale selon l'invention, comprend un ou plusieurs antigènes du SARS-CoV-2. De préférence, le ou lesdits antigènes sont spécifiques du SARS-CoV-2.

Selon un mode de réalisation, la composition immunitaire ou vaccinale permet de provoquer une réponse immunitaire contre le SARS-CoV-2, dès lors qu'elle comprend au moins un antigène du SARS-CoV-2, un micro-organisme (par exemple un virus ou une bactérie) qui peut produire au moins un antigène du SARS-CoV-2, ou bien qu'elle comprend le matériel génétique nécessaire permettant l'expression d'au moins un antigène du SARS-CoV-2 (typiquement un ARNm). Dans le premier cas, l'antigène sera en contact avec la muqueuse directement dès l'administration et dans les second et troisième cas, une période de latence pourra être attendue, le temps que l'antigène soit produit après administration de la composition. De préférence, ledit micro-organisme n'est pas pathogène *per se*, la seule réaction immunitaire qu'il peut provoquer est celle liée à l'antigène du SARS-CoV-2.

Selon un mode de réalisation particulier, les substances/antigènes permettant de provoquer une réaction immunitaire contre le SARS-CoV-2 sont choisis parmi : (i) un virus SARS-CoV-2 inactivé, (ii) un virus SARS-CoV-2 atténué, (iii) un virus SARS-CoV-2 modifié, de préférence inactivé ou atténué, exprimant ou pouvant exprimer un ou plusieurs antigènes (tels qu'une protéine virale du SARS-CoV-2) du SARS-CoV-2, (iv) un micro-organisme (e.g. bactérie ou virus, de préférence inactivé ou atténué) génétiquement modifié exprimant ou pouvant exprimer un ou plusieurs antigènes du SARS-CoV-2 (tels qu'une protéine virale du SARS-CoV-2), (v) un acide ribonucléique messager (ARNm) codant pour un ou plusieurs antigènes du SARS-CoV-2 (tels qu'une protéine virale du SARS-CoV-2), (vi) un acide désoxyribonucléique (ADN) codant pour un ou plusieurs antigènes du SARS-CoV-2 (tels qu'une protéine virale du SARS-CoV-2), (vii) une protéine virale du SARS-CoV-2 ou un ou plusieurs fragments de celle-ci, ou (viii) une protéine de fusion comprenant une protéine virale du SARS-CoV-2 ou un ou plusieurs fragments de celle-ci, ... Un fragment d'une protéine virale contient de préférence le ou les épitopes immunodominants ou biosimilaires de celle-ci. Selon un mode de réalisation, le fragment d'une protéine virale est un fragment immunogène.

La composition selon l'invention peut comprendre une ou plusieurs substances (antigènes) permettant de provoquer une réaction immunitaire contre le SARS-CoV-2. Selon un mode de réalisation, les différentes substances (antigènes) peuvent cibler différentes parties du même virus (par exemple différents épitopes sur des protéines virales), des souches différentes du même virus, des variants du même virus, voire plusieurs virus différents (vaccin combiné). La composition immunogène ou vaccinale selon l'invention peut ainsi être monovalente ou polyvalente. Une composition immunogène ou vaccinale monovalente protège contre un seul agent pathogène, alors que la composition immunogène ou vaccinale polyvalente protège contre plusieurs agents pathogènes.

Selon un mode de réalisation, l'expression « une protéine virale du SARS-CoV-2 » s'entend plus particulièrement des protéines structurales et des protéines accessoires du virus, de préférence les protéines structurales. Ainsi, selon un mode de réalisation, ladite protéine virale est choisie parmi : les protéines S, HE, M, N ou E ou les protéines ORF, plus particulièrement, ORF1a, ORF1b, ORF3, ORF6, ORF7a, ORF7b, ORF8a, ORF8b. De préférence, ladite protéine virale est choisie parmi les protéines S, HE, M, N ou E, plus particulièrement les protéines S, HE, M ou E, et encore plus particulièrement la protéine S. Un fragment immunogène de la protéine S est plus particulièrement le domaine de liaison au récepteur (RBD), la sous-unité S1 ou la région de clivage entre la sous-unité S1 et la sous-unité S2. Selon l'invention, les protéines virales s'entendent des protéines natives du virus (les protéines du virus telles que retrouvées dans la nature), des protéines mutées ou variantes par rapport aux protéines natives ou des protéines synthétisées (modifiées, mutées ou non).

La composition immunogène ou vaccinale selon l'invention est administrée chez un sujet. Selon un mode de réalisation, ladite composition immunogène ou vaccinale est utilisée dans la prévention et/ou le traitement de la Covid-19 chez l'humain. Selon un mode de réalisation, ladite composition immunogène ou vaccinale est utilisée dans la prévention et/ou le traitement de la Covid-19 chez un enfant ou un adulte, notamment un adulte. Selon l'invention, un adulte s'entend d'une personne à compter de 16 ans, de préférence une personne à compter de 18 ans. De manière encore plus préférée, l'adulte a un âge supérieur ou égal à 18 ans et jusqu'à 55 ans. Selon un mode de réalisation, ladite composition immunogène ou vaccinale peut être administrée chez un sujet ayant au moins 12 ans.

Selon un autre mode de réalisation, les applications vétérinaires de ladite composition immunogène ou vaccinale selon l'invention sont envisagées. Dans un tel cas, le sujet est ainsi un animal.

Selon un mode de réalisation, ladite composition immunogène ou vaccinale est administrée à un sujet en une quantité immunologiquement efficace. Une telle quantité peut être déterminée par le praticien. L'expression « une quantité immunologiquement efficace » s'entend d'une quantité suffisante pour être efficace sur le plan préventif et/ou thérapeutique, notamment chez un sujet ayant besoin d'une telle prévention ou d'un tel traitement. A titre d'exemple, la posologie de la composition vaccinale pourrait être entre 0,04 ml et 0,05 ml, pour chacune des 2 injections espacées d'au moins quatre semaines, de préférence entre 4 à 12 semaines, pour le vaccin à vecteur viral non réplicatif d'AstraZeneca.

Selon un mode de réalisation, ladite composition immunogène ou vaccinale est administrée à un sujet n'ayant jamais été contaminé, voire infecté, par le SARS-CoV-2 ou bien à un sujet ayant déjà été contaminé, voire infecté, par le SARS-CoV-2 (que la personne ait développé des symptômes (légers ou graves) ou ait été asymptomatique).

Selon un mode de réalisation, ladite composition immunogène ou vaccinale est administrée une fois ou plusieurs fois, de préférence une, deux ou trois fois, sur la muqueuse oculaire et/ou la muqueuse uro-génitale. Selon un mode de réalisation particulier, ladite composition immunogène ou vaccinale est administrée au moins deux fois sur la muqueuse oculaire et/ou la muqueuse uro-génitale (en d'autres termes deux doses de composition immunogène ou vaccinale sont administrées). Selon un mode de réalisation particulier, lorsque la composition immunogène ou vaccinale est administrée plusieurs fois, c'est la même muqueuse qui est ciblée : par exemple deux ou trois administrations sur la muqueuse oculaire ou deux ou trois administrations sur la muqueuse uro-génitale. De préférence, un délai d'au moins 4 semaines sépare la première et la deuxième administration, plus particulièrement un délai compris entre 4 à 12 semaines. L'expression « entre 4 à 12 semaines » signifie 4, 5, 6, 7, 8, 9, 10, 11 ou 12 semaines.

Selon un mode de réalisation, ladite composition immunogène ou vaccinale est administrée sous forme de goutte ou sous forme de lyophilisat. Typiquement, lorsque l'administration est sous forme de goutte, cela s'entend que ladite composition immunogène ou vaccinale est une formulation liquide, et lorsque l'administration est sous forme de lyophilisat, ladite composition immunogène ou vaccinale est sous forme de poudre. Ladite poudre peut être appliquée directement au niveau des muqueuses, ou bien être appliquée sur un papier filtre, un polymère, un gel ou toute autre substance ou support approprié qui sera ensuite mis en contact avec la muqueuse oculaire et/ou la muqueuse uro-génitale. La composition immunogène ou vaccinale sera ainsi transférée du papier filtre, du polymère, du gel ou toute autre substance ou support approprié vers la muqueuse oculaire et/ou uro-génitale.

Selon un mode de réalisation, ladite composition immunogène ou vaccinale est administrée à l'aide d'un applicateur, par exemple qui facilite l'instillation des gouttes (de type aide-verseur) ou de la poudre, du lyophilisat. L'applicateur peut également être une ampoule, une seringue, un papier filtre (avec par exemple un fluide ou un lyophilisat), un flacon de goutte, un applicateur de dose unique, un applicateur de type spirale ou éponge vaginale, ...

Selon un mode de réalisation, la composition immunogène selon l'invention comprend ou est constituée d'une ou plusieurs substances (antigènes) permettant de provoquer une réaction immunitaire contre le SARS-CoV-2. Selon un mode de réalisation, il peut être considéré que la composition immunogène selon l'invention est comprise dans une composition vaccinale (un vaccin). Selon un mode de réalisation, la composition immunogène ou vaccinale selon l'invention comprend (1) le vaccin à ARNm Pfizer/BioNTech, généralement dénommé Bnt162b2 ou Comirnaty^{®} ; (2) le vaccin à ARNm de Moderna, généralement dénommé Moderna mRNA-1273 ou COVID-19 Vaccine Moderna ; (3) le vaccin à vecteur viral non réplicatif d'AstraZeneca, généralement dénommé Vaxzevria^{®}, ChAdOx1-S ou COVID-19 Vaccine AstraZeneca ; (4) le vaccin à vecteur viral non réplicatif de Janssen, généralement dénommé Ad26COV2.S, JMJ Vaccine ou J & J COVID-19 ou encore (5) le vaccin à vecteur viral non réplicatif de Gamaleya, généralement dénommé Spoutnik V ou Gam-COVID-Vac.

Selon un mode de réalisation préféré, ladite composition vaccinale comprend un adjuvant et/ou un excipient.

Selon un mode de réalisation, ladite composition immunogène ou vaccinale comprend un véhicule pharmaceutiquement acceptable.

L'adjuvant et/ou l'excipient et/ou le véhicule pharmaceutiquement acceptable sont ceux classiquement utilisés. Par exemple, le véhicule pharmaceutiquement acceptable peut être un solvant ou une solution permettant de diluer la composition avant administration par voie oculaire ou sur la muqueuse uro-génitale.

Selon une mode de réalisation, une composition immunogène ou vaccinale selon l'invention, peut en outre comprendre une ou plusieurs substances additionnelles. De préférence, ladite substance additionnelle est choisie parmi :
- un conservateur,
- un marqueur, tel qu'un colorant,
- un tensioactif,
- un additif.

A titre d'exemple, un conservateur peut être un conservateur antimicrobien qui vise à empêcher la contamination microbienne de la composition immunogène ou vaccinale. Selon l'invention, le conservateur est un conservateur compatible avec les muqueuses.

Selon l'invention, un « marqueur » s'entend de toute substance mesurable ou indicatrice qui peut être administrée dans une composition immunogène ou vaccinale. Il s'agit donc d'un marqueur pharmaceutiquement acceptable. Le marqueur permet également de vérifier la quantité administrée et/ou le site d'administration, ceci afin de s'assurer d'une application sûre et efficace de la composition immunogène ou vaccinale.

Selon un mode de réalisation particulier, le marqueur permet de quantifier et/ou de visualiser l'application de ladite composition immunogène ou vaccinale sur la muqueuse oculaire et/ou la muqueuse uro-génitale. Plus précisément, la quantification s'entend de la mesure de la quantité de composition immunogène ou vaccinale administrée. Selon un mode de réalisation, le marqueur est une substance indicatrice tel qu'un colorant. A titre d'exemple les colorants de type fluorescéine ou indocyanine peuvent être utilisés. Ce marqueur sert notamment à suivre l'application de la composition immunogène ou vaccinale sur la muqueuse oculaire et/ou uro-génitale. Le colorant peut également permettre de suivre la dissolution de la composition immunogène ou vaccinale sur la muqueuse oculaire et/ou uro-génitale. La visualisation du marqueur (tel qu'un colorant) sur la muqueuse oculaire et/ou uro-génitale permet de s'assurer que la composition immunogène ou vaccinale a bien été appliquée sur la muqueuse oculaire et/ou uro-génitale, voire la quantité de composition immunogène ou vaccinale administrée.

Afin d'obtenir une antigénicité élevée et donc une réponse immunologique élevée, des substances, par exemple des tensioactifs, peuvent être ajoutées à la composition immunogène ou vaccinale, afin de prolonger le temps d'exposition sur la muqueuse oculaire et/ou uro-génitale, et éventuellement permettre de calculer le temps d'exposition de ladite composition sur la muqueuse.

D'autres substances, telles que des additifs, peuvent être ajoutées pour conférer des propriétés avantageuses à la composition immunogène ou vaccinale, par exemple une substance améliorant la pénétration de la composition immunogène ou vaccinale dans la muqueuse ou une substance permettant de prolonger le temps de contact entre la muqueuse et la composition (par exemple l'acide hyaluronique, un ou plusieurs polymères pour former un hydrogel (e.g. des carbomères), des dérivés de cellulose...).

La présente invention concerne également une méthode de prévention et/ou de traitement de la Covid-19 chez un sujet comprenant l'administration d'une composition immunogène ou vaccinale contre le SARS-CoV-2 sur la muqueuse oculaire et/ou la muqueuse uro-génitale. Plus particulièrement, la présente invention concerne une méthode pour induire une réponse immunitaire protectrice contre le SARS-CoV-2, comprenant l'administration d'une composition immunogène ou vaccinale sur la muqueuse oculaire et/ou la muqueuse uro-génitale.

La présente invention concerne également l'utilisation d'une composition immunogène ou vaccinale contre le SARS-CoV-2, pour la préparation d'un médicament destiné à la prévention et/ou au traitement de la Covid-19, et ledit médicament étant destiné à être administré sur la muqueuse oculaire et/ou la muqueuse uro-génitale.

### FIGURES

La **Figure 1** représente les résultats du groupe 1 de hamsters.
La **Figure 2** représente les résultats du groupe 2 de hamsters.

### EXEMPLES

### Exemple 1 : Exemples de compositions liquides

Le Tableau 1 ci-après, synthétise des compositions selon l'invention qui peuvent être utilisées.

**Tableau 1 : Compositions liquides**

| | **Antigène ou vaccin utilisé** | **Dose virale/concentration** | **Nombre et type d'applications** |
|---|---|---|---|
| **Composition 1** | Le vaccin à vecteur viral non réplicatif d'AstraZeneca, généralement dénommé Vaxzevria^{®}, ChAdOx1-S ou COVID-19 Vaccine AstraZeneca | 0,1*10² -10*10⁸ virus par ml | - Une ou plusieurs applications, de préférence trois |
| | | Par exemple, des doses de 0,1 mL peuvent être administrées | - Application sur un ou les deux yeux |
| **Composition 2** | Le vaccin à vecteur viral non réplicatif de Gamaleya, généralement dénommé Spoutnik V ou Gam-COVID-Vac | 0,1*10² -10*10⁸ virus par ml | - Une ou plusieurs applications, de préférence trois |
| | | Par exemple, des doses de 0,1 mL peuvent être administrées | - Application sur un ou les deux yeux |
| **Composition 3** | Protéine virale du SARS-CoV-2 (de type protéine S) | 1:10⁻¹²g/ml à 1g/ml Par exemple, des doses de 0,1 mL peuvent être administrées | - Une ou plusieurs applications, de préférence trois |
| | | | - Application sur un ou les deux yeux |
| **Composition 4** | Bactérie non pathogène génétiquement modifié pour produire une protéine virale du SARS-CoV-2 | / | - Une ou plusieurs applications, de préférence trois |
| | | | - Application sur un ou les deux yeux |

### Exemple 2 : Exemples de compositions lyophilisées

Le Tableau 2 ci-après, synthétise des compositions selon l'invention qui peuvent être utilisées.

**Tableau 2 : Compositions lyophilisées**

| | **Antigène ou vaccin utilisé** | **Nombre et type d'applications** |
|---|---|---|
| **Composition 5** | Protéine virale lyophylisée, par exemple la protéine S | - Une ou plusieurs applications, de préférence trois |
| | | - Application sur un ou les deux yeux |
| **Composition 6** | Virus lyophilisée (SARS-Cov-2, virus inactivé ou virus atténué) | - Une ou plusieurs applications, de préférence trois |
| | | - Application sur un ou les deux yeux |

### Exemple 3 : Immunisation de hamsters contre le SARS-Cov-2 par voie oculaire

Les hamsters possèdent le récepteur ACE2. Ils peuvent ainsi être contaminés par le SRAS-CoV-2 et tomber malades.

### 1. Comparaison de deux groupes de hamsters après deux injections de virus

Deux groupes de hamster ont été étudiés.

Dans le groupe 1, n=7 hamsters ont été contaminés au SRAS-CoV-2 par injection nasale de 3x10⁶ d'un virus SARS-CoV-2 (souche B.1.214) et dans le groupe 2, n=7 hamsters ont été contaminés à l'aide d'un collyre contenant 3x10⁶ virus SARS-CoV-2 (souche B.1.214).

Pendant l'observation, les hamsters du groupe 1 sont tombés malades, reconnaissables à une perte de poids significative, tandis que les hamsters du groupe 2 ne sont pas tombés malades.

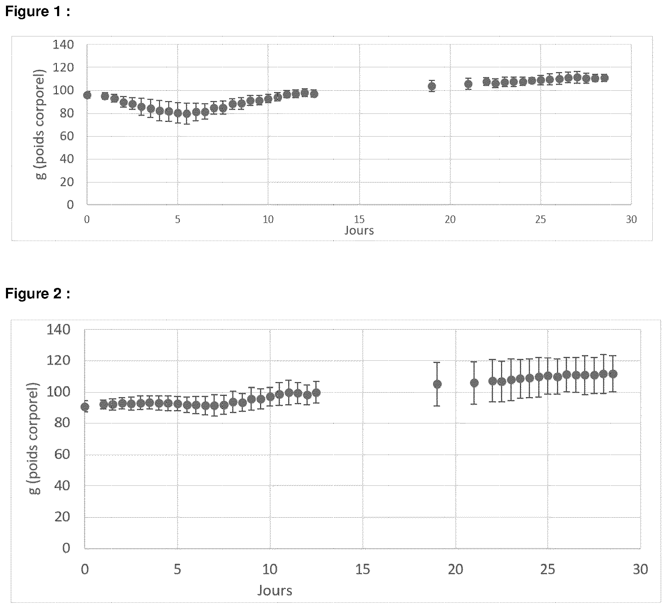

En effet, on peut observer sur la Figure 1 que les hamsters du groupe 1 ont commencé à perdre du poids à compter du jour 2 avec un maximum au jour 5 et une normalisation au jour 12, ce qui indique que les hamsters sont tombés malades suite à la contamination/infection au SRAS-CoV-2 par injection nasale. Au contraire, on peut observer sur la Figure 2 que les hamsters du groupe 2 ne perdent pas de poids après inoculation du SARS-CoV-2 par voie oculaire. Ceci indique donc que les animaux restent en bonne santé.

Après 14 jours, les deux groupes ont été à nouveau exposés à une injection nasale contenant une dose virale de 3x10⁶ de SARS-CoV-2.

Dans les deux groupes les hamsters continuent leur prise de poids (voir les Figures 1 et 2). Plus particulièrement, au jour 21, les animaux du groupe 1 ne sont pas retombés malades après l'inhalation de la deuxième dose virale de SRAS-CoV-2. Cela signifie qu'ils sont devenus immunisés en raison de la maladie développée pendant les 10 premiers jours de l'expérience.

Également, au jour 21, les hamsters du groupe 2 ne sont pas tombés malades après l'inhalation de la deuxième dose virale de SRAS-CoV-2. Cela signifie que les hamsters sont devenus immunisés grâce à la première application par voie oculaire du SRAS-CoV-2.

Les hamsters du groupe 2 ont ainsi acquis une immunité avec l'application épi-oculaire, qui, contrairement au groupe 1, a été acquise sans développer une maladie générale grave.

### 2. Etude des hamsters après une injection de virus par voie oculaire

Par ailleurs, l'étude de n=10 autres hamsters qui ont reçu une seule application par voie oculaire d'une composition contenant une dose virale de 3x10⁶ SARS-CoV-2 ont montré qu'ils ont développé une forte production d'anticorps neutralisants vis-à-vis du virus (d'au moins 1:640 dans le sérum). Ces résultats signifient que par l'application épi-oculaire du virus, les hamsters ne développent pas de maladie, et ont acquis une immunité contre le SARS-CoV-2 qui est détectable dans le sérum.

### Exemple 4 : Etude de la propagation du SARS-Cov-2 lors de l'infection de la cavité nasale

Les Inventeurs ont analysé la propagation du virus chez des hamsters qui ont été contaminés au SARS-Cov-2 via une injection nasale, tels que décrit dans l'Exemple 3.

Les Inventeurs ont ainsi constaté que dans les premières 48 heures suivant l'entrée du virus dans le nez, l'infection progresse lentement d'une infection de la cavité nasale seule aux muqueuses, avec formation d'aérosols. Ces aérosols peuvent ensuite infecter les bronches et les alvéoles, notamment lors de l'inspiration, et contaminer d'autres personnes lors d'expiration.

## Revendications

1. Composition immunogène ou vaccinale contre le SARS-CoV-2, pour son utilisation dans la prévention et/ou le traitement de la Covid-19, **caractérisée en ce qu'**elle est administrée sur la muqueuse oculaire et/ou la muqueuse uro-génitale.

2. Composition immunogène ou vaccinale selon la revendication 1, **caractérisée en ce qu'**elle est administrée sur la muqueuse oculaire.

3. Composition immunogène ou vaccinale selon l'une quelconque des revendications précédentes, **caractérisée en ce que** la composition comprend une ou plusieurs substances permettant de provoquer une réaction immunitaire contre le SARS-CoV-2.

4. Composition immunogène ou vaccinale selon la revendication 3, **caractérisée en ce que** la substance permettant de provoquer une réaction immunitaire contre le SARS-CoV-2 est choisi parmi : un virus SARS-CoV-2 inactivé, un virus SARS-CoV-2 atténué, un virus SARS-CoV-2 modifié exprimant ou pouvant exprimer un ou plusieurs antigènes du SARS-CoV-2, un micro-organisme génétiquement modifié exprimant ou pouvant exprimer un ou plusieurs antigènes du SARS-CoV-2, un acide ribonucléique messager (ARNm) codant pour un ou plusieurs antigènes du SARS-CoV-2 (tels qu'une protéine virale du SARS-CoV-2), un acide désoxyribonucléique (ADN) codant pour un ou plusieurs antigènes du SARS-CoV-2 (tels qu'une protéine virale du SARS-CoV-2), ou une protéine virale du SARS-CoV-2 ou un ou plusieurs fragments de celle-ci, une protéine de fusion comprenant une protéine virale du SARS-CoV-2 ou un ou plusieurs fragments de celle-ci.

5. Composition immunogène ou vaccinale selon la revendication 4, dans laquelle ladite protéine virale est choisie parmi : les protéines S, HE, M, N ou E ou les protéines ORF, de préférence choisie parmi les protéines S, HE, M, N ou E.

6. Composition immunogène ou vaccinale selon l'une quelconque des revendications 4 ou 5, dans laquelle ladite protéine virale est la protéine S ou un fragment de celle-ci, tel que le domaine de liaison au récepteur (RBD), la sous-unité S1 ou la région de clivage entre la sous-unité S1 et la sous-unité S2.

7. Composition immunogène ou vaccinale selon l'une quelconque des revendications précédentes, **caractérisée en ce qu'**elle est administrée sous forme de goutte ou sous forme de lyophilisat.

8. Composition immunogène ou vaccinale selon l'une quelconque des revendications précédentes, **caractérisée en ce qu'**elle est administrée à l'aide d'un applicateur.

9. Composition immunogène ou vaccinale selon l'une quelconque des revendications précédentes, pour son utilisation dans la prévention et/ou le traitement de la Covid-19 chez un animal ou l'humain, notamment un adulte.

10. Composition immunogène ou vaccinale selon l'une quelconque des revendications précédentes, **caractérisée en ce qu'**elle comprend en outre une ou plusieurs substances additionnelles.

11. Composition immunogène ou vaccinale selon la revendication 10, **caractérisée en ce que** la substance additionnelle est choisi parmi :
- un conservateur,
- une substance indicatrice, telle qu'un colorant,
- un tensioactif,
- un additif.
